# EUROPEAN PATENT APPLICATION

(11) **EP 3 437 641 A1**
(43) Date of publication of application: **06.02.2019**
(21) Application number: 17774997.5
(22) Date of filing: 28.03.2017
(51) Int. Cl.: A61K 31/4172, A61P 1/04

(54) **INFLAMMATORY INTESTINAL DISEASE THERAPEUTIC AGENT**

(30) Priority: 29.03.2016 JP 2016064993
(71) Applicant: Daiichi Sankyo Company, Limited, Tokyo 103-8426 (JP)
(72) Inventor: NOGUCHI, Kengo, Tokyo 103-8426 (JP); ITO, Yusuke, Tokyo 103-8426 (JP)
(74) Representative: Nieuwenhuys, William Francis
(86) International application number: PCT/JP2017/012497
(87) International publication number: WO 2017/170460

(57) **Abstract**

A therapeutic agent for inflammatory bowel diseases comprising as an active ingredient a compound represented by the formula (I) wherein R represents a hydrogen atom or a [1-(isobutyryloxy)ethoxy]carbonyl group or the like, or a pharmacologically acceptable salt thereof.

## Description

### Technical Field

The present invention relates to a therapeutic agent for inflammatory bowel diseases containing a TAFIa inhibitor as an active ingredient, and a method for treating an inflammatory bowel disease, characterized by administering a TAFIa inhibitor.

### Background Art

Inflammatory bowel disease (IBD) is a generic term for diseases causing chronic inflammation or ulceration in the mucosa of the large intestine and small intestine, and representative diseases are ulcerative colitis and Crohn's disease. Ulcerative colitis is a diffuse nonspecific inflammation of unknown cause in which ulcer or erosion occurs mainly in the large intestinal mucosa, and is a disease exhibiting various systemic symptoms including bloody diarrhea. Crohn's disease is a disease of unknown cause inducing discontinuous chronic granulomatous inflammation in the entire gastrointestinal tract from the oral cavity through to the anus. Other examples of inflammatory bowel diseases include intestinal Behcet's disease, hemorrhagic rectal ulcer and pouchitis or the like, but the direct cause of any of these diseases is still unknown (Non Patent Literature 1). For the treatment of inflammatory bowel diseases, drugs such as an immunosuppressive drug, a steroid drug, salazosulfapyridine, mesalazine or the like are used, but they are incomplete in terms of efficacy and safety. Therefore, it is required to develop a drug that is more effective and has few side effects (Non Patent Literature 2 and 3).
Non Patent Literature 4 and 5 report that the level of activated thrombin-activatable fibrinolysis inhibitor (hereinafter referred to as "TAFIa") in blood is increased in patients with inflammatory bowel diseases. Patent Literature 1 to 7 disclose a group of compounds exhibiting TAFIa inhibitory activity, which are useful as therapeutic agents for thrombosis and embolism. However, there is no report that a TAFIa inhibitor is effective for treatment of inflammatory bowel diseases.

### Citation List

### Patent Literature

Patent Literature 1: International Publication WO 2002/014285
Patent Literature 2: International Publication WO 2003/061652
Patent Literature 3: International Publication WO 2003/061653
Patent Literature 4: International Publication WO 2005/105781
Patent Literature 5: International Publication WO 2003/013526
Patent Literature 6: International Publication WO 2011/115064
Patent Literature 7: International Publication WO 2011/115065

### Non Patent Literature

Non Patent Literature 1: New Engl J Med, 2002, 347, 417 - 429
Non Patent Literature 2: Am J Gastroenterol, 2001, 96, 1977 - 1997
Non Patent Literature 3: Nucl Med Commun, 2005, 26, 649 - 655
Non Patent Literature 4: Am J Gastroenterol, 2004, 99, 1966 - 1970
Non Patent Literature 5: Journal of Crohn's and Colitis, 2012, 6, 13 - 20

### Summary of Invention

### Technical Problem

The object of the present invention is to provide a therapeutic agent for inflammatory bowel diseases containing a TAFIa inhibitor as an active ingredient, and a method for treating an inflammatory bowel disease, characterized by administering a TAFIa inhibitor.

### Solution to Problem

The present inventors have conducted studies with the aim of examining the use of a TAFIa inhibitor as a therapeutic agent for inflammatory bowel diseases. As a result, they have found that the TAFIa inhibitor is effective for treatment of inflammatory bowel diseases. Specifically, the present invention includes the following aspects:
(1) A therapeutic agent for inflammatory bowel diseases comprising as an active ingredient a compound represented by the formula (I): 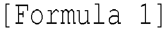 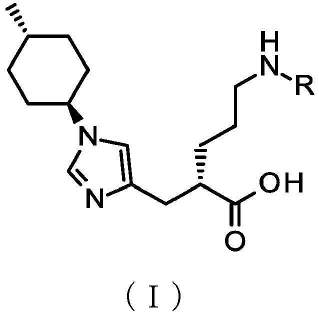 wherein R represents a hydrogen atom, a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, or a (1-acetoxyethoxy)carbonyl group,
   or a pharmacologically acceptable salt thereof.
(2) The therapeutic agent for inflammatory bowel diseases according to (1), wherein R represents a hydrogen atom.
(3) The therapeutic agent for inflammatory bowel diseases according to (2), wherein the pharmacologically acceptable salt is p-toluenesulfonate.
(4) The therapeutic agent for inflammatory bowel diseases according to (1), wherein R represents a [1-(isobutyryloxy)ethoxy]carbonyl group.
(5) The therapeutic agent for inflammatory bowel diseases according to (4), wherein R represents a [(1R)-1-(isobutyryloxy)ethoxy]carbonyl group.
(6) The therapeutic agent for inflammatory bowel diseases according to any one of (1) to (5), wherein the inflammatory bowel disease is ulcerative colitis, Crohn's disease, intestinal Behcet's disease, hemorrhagic rectal ulcer or pouchitis.
(7) The therapeutic agent for inflammatory bowel diseases according to any one of (1) to (5), wherein the inflammatory bowel disease is ulcerative colitis.
(8) A method for treating an inflammatory bowel disease, characterized by administering a compound represented by the formula (I): 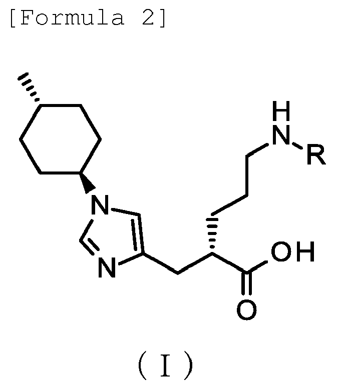 wherein R represents a hydrogen atom, a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, or a (1-acetoxyethoxy)carbonyl group, or a pharmacologically acceptable salt thereof.
(9) The method for treating an inflammatory bowel disease according to (8), wherein R represents a hydrogen atom.
(10) The method for treating an inflammatory bowel disease according to (9), wherein the pharmacologically acceptable salt is p-toluenesulfonate.
(11) The method for treating an inflammatory bowel disease according to (8), wherein R represents a [1-(isobutyryloxy)ethoxy]carbonyl group.
(12) The method for treating an inflammatory bowel disease according to (11), wherein R represents a [(1R)-1-(isobutyryloxy)ethoxy]carbonyl group.
(13) The method for treating an inflammatory bowel disease according to any one of (8) to (12), wherein the inflammatory bowel disease is ulcerative colitis, Crohn's disease, intestinal Behcet's disease, hemorrhagic rectal ulcer or pouchitis.
(14) The method for treating an inflammatory bowel disease according to any one of (8) to (12), wherein the inflammatory bowel disease is ulcerative colitis.

### Advantageous Effects of Invention

According to the present invention, a therapeutic agent for inflammatory bowel diseases containing a TAFIa inhibitor as an active ingredient, and a method for treating an inflammatory bowel disease characterized by administering a TAFIa inhibitor, can be provided.

### Brief Description of Drawings

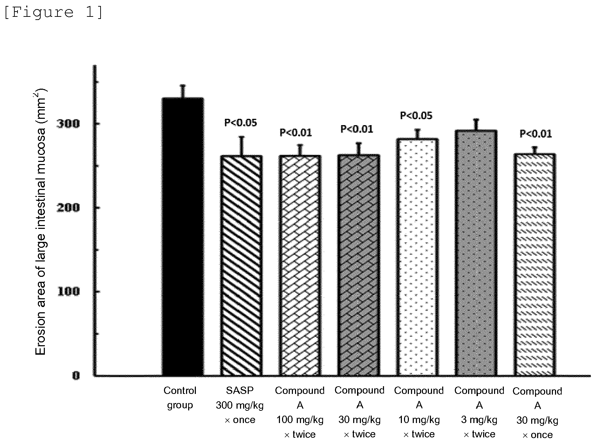

Figure 1 shows the results of the erosion area of the large intestinal mucosa measured in the control group; the oral administration group of 300 mg/kg of SASP, once a day; the oral administration group of 100 mg/kg of compound A, twice a day; the oral administration group of 30 mg/kg of compound A, twice a day; the oral administration group of 10 mg/kg of compound A, twice a day; the oral administration group of 3 mg / kg of compound A, twice a day; and the oral administration group of 30 mg/kg of compound A, once a day.

### Description of Embodiments

Examples of the TAFIa inhibitor to be used in the present invention include carboxypeptidase inhibitor from potato tuber, 5-amino-2-[(1-cyclohexyl-1H-imidazol-4-yl)methyl]valeric acid, 5-amino-2-{[1-(4-methylcyclohexyl)-1H-imidazol-4-yl]methyl}valeric acid, 5-amino-2-{[1-(4-ethylcyclohexyl)-1H-imidazol-4-yl]methyl}valeric acid, 5-amino-2-{[1-(3-ethylcyclobutyl)-1H-imidazol-4-yl]methyl}valeric acid, 5-amino-2-{[1-(3-methylcyclobutyl)-1H-imidazol-4-yl]methyl}valeric acid, 5-amino-2-({1-[(1R,3s,5S)-bicyclo[3.1.0]hexan-3-yl]-1H-imidazol-4-yl}methyl)valeric acid, 5-amino-2-{[1-(4-hydroxycyclohexyl)-1H-imidazol-4-yl]methyl}valeric acid, 5-amino-2-{[1-(4-hydroxy-4-methylcyclohexyl)-1H-imidazol-4-yl]methyl}valeric acid, 5-amino-2-{[1-(3-methylcyclohexyl)-1H-imidazol-4-yl]methyl}valeric acid, 5-amino-2-[(1-cycloheptyl-1H-imidazol-4-yl)methyl]valeric acid, 5-amino-2-({1-[exo-bicyclo[2.2.1]heptan-2-yl]-1H-imidazol-4-yl}methyl)valeric acid, 5-amino-2-({1-[endo-bicyclo[2.2.1]heptan-2-yl]-1H-imidazol-4-yl}methyl)valeric acid, 2-[(1-adamantan-2-yl-1H-imidazol-4-yl)methyl]-5-aminovaleric acid, 5-amino-2-{[1-(4-phenoxycyclohexyl)-1H-imidazol-4-yl]methyl}valeric acid, benzyl 5-amino-2-{[1-(4-methylcyclohexyl)-1H-imidazol-4-yl]methyl}valerate, 2-{[1-(4-methylcyclohexyl)-1H-imidazol-4-yl]methyl}-5-(L-phenylalanylamino)valeric acid, 2-{[1-(4-methylcyclohexyl)-1H-imidazol-4-yl]methyl}-5-(L-norleucylamino)valeric acid, 2-{[1-(4-methylcyclohexyl)-1H-imidazol-4-yl]methyl}-5-({[(5-methyl-2-oxo-1,3-dioxol-4-yl)methoxy]carbonyl}amino)valeric acid, 5-({[1-(isobutyryloxy)ethoxy]carbonyl}amino)-2-{[1-(4-methylcyclohexyl)-1H-imidazol-4-yl]methyl}valeric acid, 1-[(isopropoxycarbonyl)oxylethyl 5-({[1-(isobutyryloxy)ethoxy]carbonyl}amino)-2-{[1-(4-methylcyclohexyl)-1H-imidazol-4-yl]methyl}valerate, 5-({[1-(2,2-dimethylpropanoyloxy)ethoxy]carbonyl}amino)-2-{[1-(4-methylcyclohexyl)-1H-imidazol-4-yl]methyl}valeric acid, 5-[({1-[(cyclohexylcarbonyl)oxy]ethoxy}carbonyl)amino]-2-{[1-(4-methylcyclohexyl)-1H-imidazol-4-yl]methyl}valeric acid, 2-(2-aminoethoxy)-3-[1-(4-methylcyclohexyl)-1H-imidazol-4-yl]propionic acid, 2-[(1R)-2-amino-1-methylethoxy]-3-[1-(4-methylcyclohexyl)-1H-imidazol-4-yl]propionic acid, 2-[(3S)-3-aminopyrrolidin-1-yl]-3-[1-(4-methylcyclohexyl)-1H-imidazol-4-yl]propion, (2S)-2-{[1-(trans-4-methylcyclohexyl)-1H-imidazol-4-yl]methyl}-5-({[(5-methyl-2-oxo-1,3-dioxol-4-yl)methoxy]carbonyl}amino)valeric acid, (2S)-5-({[1-(isobutyryloxy)ethoxy]carbonyl}amino)-2-{[1-(trans-4-methylcyclohexyl)-1H-imidazol-4-yl]methyl}valeric acid, 1-[(isopropoxycarbonyl)oxy]ethyl(2S)-5-({[1-(isobutyryloxy)ethoxy]carbonyl}amino)-2-{[1-(trans-4-methylcyclohexyl)-1H-imidazol-4-yl]methyl}valerate, (2S)-5-({[1-(2,2-dimethylpropanoyloxy)ethoxy]carbonyl}amino)-2-{[1-(trans-4-methylcyclohexyl)-1H-imidazol-4-yl]methyl}valeric acid, (2S)-5-[({1-[(cyclohexylcarbonyl)oxy]ethoxy}carbonyl)amino]-2-{[1-(trans-4-methylcyclohexyl)-1H-imidazol-4-yl]methyl}valeric acid, (2S)-5-{[(1-acetoxyethoxy)carbonyl]amino}-2-{[1-(trans-4-methylcyclohexyl)-1H-imidazol-4-yl]methyl}valeric acid, (2S)-2-{[1-(trans-4-methylcyclohexyl)-1H-imidazol-4-yl]methyl}-5-[({[(2-methylpropanoyl)oxy]methoxy}carbonyl)amino]valeric acid, (2S)-5-[({[(2,2-dimethylpropanoyl)oxy]methyloxy}carbonyl)amino]-2-{ [1-(trans-4-methylcyclohexyl)-1H-imidazol-4-yl]methyl}valeric acid, (2S)-5-[({[(cyclohexylcarbonyl)oxy]methoxylcarbonyl)amino]-2-{[1-(trans-4-methylcyclohexyl)-1H-imidazol-4-yl]methyl}valeric acid, (2S)-5-({[(acetyloxy)methoxy]carbonyl}amino)-2-{[1-(trans-4-methylcyclohexyl)-1H-imidazol-4-yl]methyl}valeric acid, (2S)-5-({[(1R)-1-(isobutyryloxy)ethoxy]carbonyl}amino)-2-{[1-(trans-4-methylcyclohexyl)-1H-imidazol-4-yl]methyl}valeric acid, 2-(3-aminopropyl)-1-(1-phenyl-1H-imidazol-4-yl)cyclopropanecarboxylic acid, 2-(3-aminopropyl)-1-[1-(3,3-dimethylbutyl)-1H-imidazol-4-yl]cyclopropanecarboxylic acid, 2-(3-aminopropyl)-1-[1-(trans-4-methylcyclohexyl)-1H-imidazol-4-yl]cyclopropanecarboxylic acid, 2-(3-aminopropyl)-1-(1-pyridin-2-yl-1H-imidazol-4-yl)cyclopropanecarboxylic acid, 2-(3-amino-2-methylpropyl)-1-(1-phenyl-1H-imidazol-4-yl)cyclopropanecarboxylic acid, 2-(3-aminopropyl)-1-[1-(5-methylpyridin-2-yl)-1H-imidazol-4-yl]cyclopropanecarboxylic acid, 2-[(2-aminomethyl)butyl]-1-(1-phenyl-1H-imidazol-4-yl)cyclopropanecarboxylic acid, 2-[(2R)-3-amino-2-methylpropyl]-1-(1-pyridin-2-yl-1H-imidazol-4-yl)cyclopropanecarboxylic acid, 2-[(2R)-3-amino-2-methylpropyl]-1-[1-(5-methylpyridin-2-yl)-1H-imidazol-4-yl]cyclopropanecarboxylic acid, 2-[(2R)-2-(aminomethyl)butyl]-1-(1-pyridin-2-yl-1H-imidazol-4-yl)cyclopropanecarboxylic acid, (1R,2S)-2-(3-aminopropyl)-1-(1-phenyl-1H-imidazol-4-yl)cyclopropanecarboxylic acid, (1R,2S)-2-(3-aminopropyl)-1-(1-pyridin-2-yl-1H-imidazol-4-yl)cyclopropanecarboxylic acid, (1R,2S)-2-[(2R)-3-amino-2-methylpropyl]-1-(1-pyridin-2-yl-1H-imidazol-4-yl)cyclopropanecarboxylic acid, (1R,2S)-2-[(2R)-3-amino-2-methylpropyl]-1-[1-(5-methylpyridin-2-yl)-1H-imidazol-4-yl]cyclopropanecarboxylic acid, (1R,2S)-2-[(2R)-2-(aminomethyl)butyl]-1-(1-pyridin-2-yl-1H-imidazol-4-yl)cyclopropanecarboxylic acid, SAR-126119, SAR-104772, UK-396082, BX-528, AZD-9684, EF-6265/MN-462, or a pharmacologically acceptable salt thereof; preferably (2S)-5-amino-2-{[1-(trans-4-methylcyclohexyl)-1H-imidazol-4-yl]methyl}valeric acid, (2S)-2-{ [1-(trans-4-methylcyclohexyl)-1H-imidazol-4-yl]methyl}-5-({[(5-methyl-2-oxo-1,3-dioxol-4-yl)methoxy]carbonyl}amino)valeric acid, (2S)-5-({[1-(isobutyryloxy)ethoxy]carbonyl}amino)-2-{[1-(trans-4-methylcyclohexyl)-1H-imidazol-4-yl]methyl}valeric acid, (2S)-5-({[1-(2,2-dimethylpropanoyloxy)ethoxy]carbonyl}amino)-2-{[1-(trans-4-methylcyclohexyl)-1H-imidazol-4-yl]methyl}valeric acid, (2S)-5-[({1-[(cyclohexylcarbonyl)oxy]ethoxy}carbonyl)amino]-2-{[1-(trans-4-methylcyclohexyl)-1H-imidazol-4-yl]methyl}valeric acid, (2S)-5-{[(1-acetoxyethoxy)carbonyl]amino}-2-{[1-(trans-4-methylcyclohexyl)-1H-imidazol-4-yl]methyl}valeric acid, or a pharmacologically acceptable salt thereof; and more preferably (2S)-5-amino-2-{[1-(trans-4-methylcyclohexyl)-1H-imidazol-4-yl]methyl}valeric acid, (2S)-5-({[1-(isobutyryloxy)ethoxy]carbonyl}amino)-2-{[1-(trans-4-methylcyclohexyl)-1H-imidazol-4-yl]methyl}valeric acid, or a pharmacologically acceptable salt thereof; still more preferably (2S)-5-amino-2-{[1-(trans-4-methylcyclohexyl)-1H-imidazol-4-yl]methyl}valeric acid, (2S)-5-({[(1R)-1-(isobutyryloxy)ethoxy]carbonyl}amino)-2-{[1-(trans-4-methylcyclohexyl)-1H-imidazol-4-yl]methyl}valeric acid, or a pharmacologically acceptable salt thereof; and still further more preferably (2S)-5-amino-2-{[1-(trans-4-methylcyclohexyl)-1H-imidazol-4-yl]methyl}valeric acid p-toluenesulfonate.

The preferred TAFIa inhibitor to be used in the present invention can be represented by the formula (I): 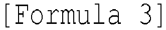 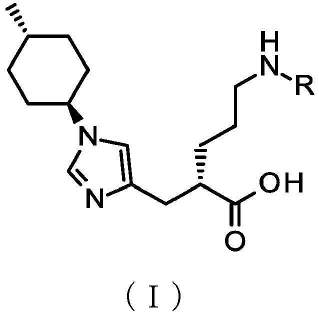 wherein R represents a hydrogen atom, a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, or a (1-acetoxyethoxy)carbonyl group.

The compounds represented by the formula (I) wherein R represents a hydrogen atom, i.e., (2S)-5-amino-2-{[1-(trans-4-methylcyclohexyl)-1H-imidazol-4-yl]methyl}valeric acid is described in Example 15 (2S-isomer) in International Publication WO 2011/115064.

The compound represented by the formula (I) wherein R represents a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, a (1-acetoxyethoxy)carbonyl group, and a [(1R)-1-(isobutyryloxy)ethoxy]carbonyl group, i.e., (2S)-2-{[1-(trans-4-methylcyclohexyl)-1H-imidazol-4-yl]methyl}-5-({[(5-methyl-2-oxo-1,3-dioxol-4-yl)methoxy]carbonyl}amino)valeric acid, (2S)-5-({[1-(isobutyryloxy)ethoxy]carbonyl}amino)-2-{[1-(trans-4-methylcyclohexyl)-1H-imidazol-4-yl]methyl}valeric acid, (2S)-5-({[1-(2,2-dimethylpropanoyloxy)ethoxy]carbonyl}amino)-2-{[1-(trans-4-methylcyclohexyl)-1H-imidazol-4-yl]methyl}valeric acid, (2S)-5-[({1-[(cyclohexylcarbonyl)oxy]ethoxy}carbonyl)amino]-2-{[1-(trans-4-methylcyclohexyl)-1H-imidazol-4-yl]methyl}valeric acid, (2S)-5-{[(1-acetoxyethoxy)carbonyl]amino}-2-{[1- (trans-4-methylcyclohexyl)-1H-imidazol-4-yl]methyl}valeric acid, and (2S)-5-({[(1R)-1-(isobutyryloxy)ethoxy]carbonyl}amino)-2-{[1-(trans-4-methylcyclohexyl)-1H-imidazol-4-yl]methyl}valeric acid are prodrugs that are converted in vivo into an active ingredient, (2S)-5-amino-2-{[1-(trans-4-methylcyclohexyl)-1H-imidazol-4-yl]methyl}valeric acid, by reaction with an enzyme, gastric acid or the like. These compounds are described in Examples 19, 20, 22, 23, 27 and 32 in International Publication WO 2011/115064.

Among the pharmacologically acceptable salts of a TAFIa inhibitor to be used in the present invention, examples of acid addition salts include a hydrohalide such as a hydrofluoride, a hydrochloride, a hydrobromide and a hydroiodide ; an inorganic acid salt such as a nitrate, a perchlorate, a sulfate and a phosphate; a lower alkane sulfonate such as a methanesulfonate, a trifluoromethanesulfonate and a ethanesulfonate; an aryl sulfonate such as a benzenesulfonate and a p-toluenesulfonate; an organic acid salt such as an acetate, a malate, a fumarate, a succinate, a citrate, a tartrate, an oxalate and a maleate; and an amino acid salt such as an ornithinate, a glutamate and an aspartate. When R in the compound represented by the formula (I) is a hydrogen atom, the pharmacologically acceptable salt thereof is preferably a hydrohalide or an aryl sulfonate, more preferably a hydrochloride, a benzensulfonate or a p-toluenesulfonate, still more preferably a benzensulfonate or a p-toluenesulfonate, and particularly preferably a p-toluenesulfonate. (2S)-5-amino-2-{[1-(trans-4-methylcyclohexyl)-1H-imidazol-4-yl]methyl}valeric acid p-toluenesulfonate anhydride is described in Example 40 in International Publication WO 2011/115064.

Examples of base addition salts include an alkali metal salt such as a sodium salt, a potassium salt and a lithium salt; an alkaline earth metal salt such as a calcium salt and a magnesium salt; an inorganic salt such as an ammonium salt; an organic amine salt such as a dibenzylamine salt, a morpholine salt, a phenylglycine alkyl ester salt, an ethylenediamine salt, an N-methylglucamine salt, a diethylamine salt, a triethylamine salt, a cyclohexylamine salt, a dicyclohexylamine salt, an N, N'-dibenzylethylenediamine salt, a diethanolamine salt, an N-benzyl-N-(2-phenylethoxy)amine salt, a piperazine salt, a tetramethylammonium salt, a tris(hydroxymethyl)aminomethane salt; and an amino acid salt such as arginine salt,

The TAFIa inhibitor or pharmacologically acceptable salt thereof to be used in the present invention may also be present as a solvate thereof, and such a solvate is included in the compound to be used or its salt. The solvate is not particularly limited as long as it is pharmacologically acceptable. Specifically, it is preferably a hydrate, an ethanolate or the like, and more preferably a hydrate.

The TAFIa inhibitor or pharmacologically acceptable salt thereof to be used in the present invention may be in the form of an oral formulation or a parenteral formulation.
Examples of the oral formulation include a tablet, a pill, a powder, a granule, a capsule, a solution, a suspension, an emulsion, a syrup and an elixir. The medicine in such a form is usually prepared as a pharmaceutical composition which comprises the TAFIa inhibitor or pharmacologically acceptable salt thereof to be used in the present invention as a main ingredient mixed with a pharmaceutically acceptable additive such as a diluent, an excipient or a carrier. In addition, the pharmaceutical composition can be prepared conventionally by using, as needed, an additive appropriately selected from any suitable pharmaceutically acceptable binder, disintegrator, lubricant, swelling agent, swelling aid, coating agent, plasticizer, stabilizer, antiseptic, antioxidant, colorant, solubilizing agent, suspending agent, emulsifier, sweetener, preservative, buffer, wetting agent and the like.

Examples of the parenteral formulation include an injection, an ointment, a gel, a cream, a poultice, a patch, an aerosol, an inhalant, a spray, an eye drop, a nasal drop, a suppository and an inhalant. The medicine in such a form is usually prepared as a pharmaceutical composition which comprises the TAFIa inhibitor or pharmacologically acceptable salt thereof to be used in the present invention as a main ingredient mixed with a pharmaceutically acceptable additive such as a diluent, an excipient or a carrier. In addition, the pharmaceutical composition can be prepared conventionally by using, as needed, an additive appropriately selected from any suitable pharmaceutically acceptable stabilizer, antiseptic, solubilizing agent, humectant, preservative, antioxidant, flavoring agent, gelling agent, neutralizer, buffer, tonicity adjusting agent, surfactant, colorant, thickener, wetting agent, filler, absorption enhancer, suspending agent, binder and the like.

A single dose of the TAFIa inhibitor or pharmacologically acceptable salt thereof to be used in the present invention is 0.01 to 5000 mg, preferably 0.1 to 1000 mg and more preferably 1 to 200 mg.

### Examples

Hereinafter, the present invention will be specifically described with reference to examples thereof, but is not limited thereto by any means.

The test of drug efficacy of a TAFIa inhibitor on dextran sodium sulfate-induced ulcerative colitis in rats will be described below. This test was conducted by commissioning to NISSEI BILIS Co.,Ltd.

### 1. Test Procedure

### 1-1. Preparation of dosing solution

### (1) Test substance

A TAFIa inhibitor, (2S)-5-amino-2-{[1-(trans-4-methylcyclohexyl)-1H-imidazol-4-yl]methyl}valeric acid p-toluenesulfonate anhydride (hereinafter referred to as "compound A") was dissolved in a 0.5% aqueous sodium carboxymethylcellulose solution (hereinafter referred to as "CMC-Na") to prepare a dosing solution for oral administration of 0.6, 2, 6 and 20 mg/mL. The dosage was determined by conversion into a free form with a conversion factor of 0.6301.
The chemical structure of compound A is as follows:

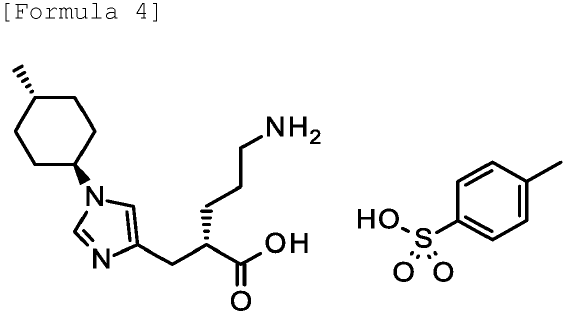

### (2) Positive control substance

Salazosulfapyridine (hereinafter referred to as "SASP"), a conventional therapeutic agent for inflammatory bowel diseases, was dissolved in a 0.5% aqueous CMC-Na solution to prepare a dosing solution of 60 mg/mL.

The chemical structure of SASP is as follows:

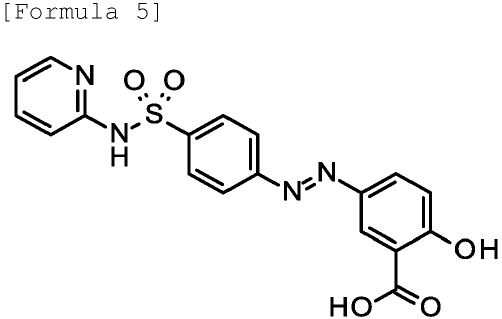

### (3) Control substance

A 0.5% aqueous CMC-Na solution was used.

### 1-2. Induction of ulcerative colitis

7-Week old male LEW/SsN Slc rats (Japan SLC, Inc.) were allowed to drink 3% dextran sodium sulfate (hereinafter referred to as "DSS") freely to create ulcerative colitis models. Individuals meeting selection criteria for model animals shown in 1-3 below were selected followed by switching to free drinking of 1% aqueous DSS solution to maintain the ulcerative colitis models.

### 1-3. Selection criteria of individuals

The day when hematochezia was observed in 90% or more of individuals after the start of drinking of a 3% DSS aqueous solution was designated as a selection day, and the individuals were tested according to the following test items. Thereafter, animals that met the selection criteria were selected.

### <Test Items>

1) Hematochezia: Observed every morning from after the start of drinking a 3% DSS aqueous solution until the selection day.
2) Body weight: Measured from the day when hematochezia was observed in 70% or more of individuals
3) Hemoglobin concentration: Measured with a hemoglobin analyzer (HemoCue Hb 201 + photometer, AMCO Incorporated) by collecting approximately 10 µL of blood from the tail vein on the selection day. Note that the hemoglobin concentration was measured whether or not DSS treatment was conducted.

### <Selection criteria>

1) Hematochezia: Observed for consecutive 2 days or more including the selection day.
2) Body weight: The body weight on the selection day not decreased by 20 g or more compared with that on the day before.
3) Hemoglobin concentration: 10 g/dL or more.

### 1-4. Grouping procedure

The tests were conducted in two separate groups (the first half: 5 animals/group; the second half: 5 animals/group). The untreated individuals and the individuals meeting the above selection criteria were selected as adopted animals, and grouped into groups of 5 animals/group with the body weight as a main parameter and the hemoglobin concentration as a secondary parameter, according to stratified randomized assignment so as not to cause a difference in the average value among the groups (the total of two tests: 10 animals/group).

### 1-5. Composition of test groups

**[Table 1]**

| Test group | Dose (mg/kg) | Administration route | Frequency of administration | Number of animals |
|---|---|---|---|---|
| Untreated group | - | | - | 10 |
| Control group | - | | Twice/day | 10 |
| SASP | 300 | | Twice/day* | 10 |
| Compound A | 100 | | Twice/day | 10 |
| Compound A | 30 | | Twice/day | 10 |
| Compound A | 10 | | Twice/day | 10 |
| Compound A | 3 | | Twice/day | 10 |
| Compound A | 30 | | Twice/day* | 10 |

| | | | | |
|---|---|---|---|---|
| * Afternoon administration is a vehicle administration. | | | | |

### 1-6. Administration

Administration route: Oral administration
Administration volume: 5 mL/kg
Administration method: Administration was conducted by using a polypropylene disposable syringe and a probe for oral administration. Note that the administration places were different in the morning and the afternoon.
Dosing period: Administration was conducted twice a day at approximately the same time of the day (the first time: 8:00 to 11:00; the second time: 15:00 to 18:00) repeatedly for 14 days. Administration was conducted by masking the test groups (that is, individual identification numbers were recorded in the animal cards, and administration was conducted in a blinded manner). Note that the administration on the selection day (the first day of administration) was conducted only once in the afternoon.

### 1-7. Preparation of large intestine specimens

Each of animals was fasted from the end of the final administration. On the day of dissection, approximately 3 mL of blood was collected from the abdominal aorta under anesthesia with pentobarbital sodium (30 mg/kg, i.p.) by using a polypropylene disposable syringe and an injection needle. Immediately after blood collection, the large intestine (the colon and the rectum) was isolated and immersed in physiological saline kept warm at approximately 37°C, and the length of the large intestine was measured in a relaxed state. Thereafter, a 10% neutral buffered formalin solution was infused into the large intestine, and the large intestine was immersed in physiological saline with the lumen expanded and temporarily fixed for 1 hour or more. After temporary fixation, the large intestine was dissected along the mesentery attachment site, immersed in a stretched state in a 10% neutral buffered formalin solution, and fixed for 1 week or more. The fixed large intestine specimen was washed with running water for approximately 5 minutes, further with distilled water three times, and then immersed in a 3% aqueous acetic acid solution for approximately 5 minutes as a pretreatment. Thereafter, the specimen was stained for approximately 20 minutes by immersing it in a 1% Alcian blue solution (dissolved in a 3% aqueous acetic acid solution), and then washed with a 3% aqueous acetic acid solution four times until Alcian blue did not elute off.

### 1-8. Measurement of erosion area of the large intestinal mucosa

The large intestine specimen stained with a 1% Alcian blue solution was photographed. The photograph was captured on an image analysis device (general-purpose image processor WinROOF, Version 5.7, MITANI CORPORATION), and the area of deep blue part was measured. This measurement value was used as the erosion area.

### 1-9. Statistical processing of data

The erosion area was expressed as mean ± standard error. A significant difference test between the control group and each group was conducted by a homoscedasticity test (F-test) followed by Student's t test in the case of homoscedasticity and by Aspin-Welch's t test in the case of heteroscedasticity (significance level: 5%).

### 2. Test results

In the untreated group, no erosion of the large intestinal mucosa was observed.
The erosion area of the control group (0.5% CMC-Na) was 330.3 ± 15.4 mm².
In the case of administering 300 mg/kg of SASP orally once a day, the erosion area was 261.6 ± 22.7 mm² (inhibition rate: 20.8%). A significant reduction in the erosion area was observed compared to the control group (Student's t test: P < 0.05).
In the case of administering 100 mg/kg of compound A orally twice a day, the erosion area was 261.9 ± 12.7 mm² (inhibition rate: 20.7%). A significant reduction in the erosion area was observed compared to the control group (Student's t test: P < 0.01).
In the case of administering 30 mg/kg of compound A orally twice a day, the erosion area was 262.89 ± 13.9 mm² (inhibition rate: 20.4%). A significant reduction in the erosion area was observed compared to the control group (Student's t test: P < 0.01).
In the case of administering 10 mg/kg of compound A orally twice a day, the erosion area was 281.6 ± 11.1 mm² (inhibition rate: 14.7%). A significant reduction in the erosion area was observed compared to the control group (Student's t test: P < 0.05).
In the case of administering 3 mg/kg of compound A orally twice a day, the erosion area was 291.8 ± 13.1 mm² (inhibition rate: 11.7%). No significant difference was observed as compared with the control group.
In the case of administering 30 mg/kg of compound A orally once a day, the erosion area was 264.0 ± 7.9 mm² (inhibition rate: 20.1%). A significant reduction in the erosion area was observed compared to the control group (Student's t test: P < 0.01).
The above measurement results of the erosion area of the large intestinal mucosa are shown in Figure 1.

### 3. Discussion

Compound A exhibited a significant reduction effect of the erosion area in the rat ulcerative colitis model. From the above results, it was shown that a TAFIa inhibitor is useful as a therapeutic agent for inflammatory bowel diseases.

### Industrial Applicability

According to the present invention, it was shown that a TAFIa inhibitor is useful as a therapeutic agent for inflammatory bowel diseases. Therefore, the present invention can provide a therapeutic agent for inflammatory bowel diseases containing a TAFIa inhibitor as an active ingredient, and a method for treating an inflammatory bowel disease, characterized by administering a TAFIa inhibitor.

## Claims

1. A therapeutic agent for inflammatory bowel diseases comprising as an active ingredient a compound represented by the formula (I): 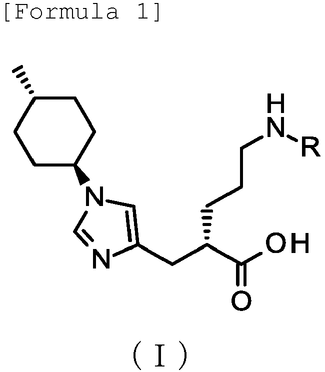 wherein R represents a hydrogen atom, a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, or a (1-acetoxyethoxy)carbonyl group,
or a pharmacologically acceptable salt thereof.

2. The therapeutic agent for inflammatory bowel diseases according to claim 1, wherein R represents a hydrogen atom.

3. The therapeutic agent for inflammatory bowel diseases according to claim 2, wherein the pharmacologically acceptable salt is p-toluenesulfonate.

4. The therapeutic agent for inflammatory bowel diseases according to claim 1, wherein R represents a [1-(isobutyryloxy)ethoxy]carbonyl group.

5. The therapeutic agent for inflammatory bowel diseases according to claim 4, wherein R represents a [(1R)-1-(isobutyryloxy)ethoxy]carbonyl group.

6. The therapeutic agent for inflammatory bowel diseases according to any one of claims 1 to 5, wherein the inflammatory bowel disease is ulcerative colitis, Crohn's disease, intestinal Behcet's disease, hemorrhagic rectal ulcer or pouchitis.

7. The therapeutic agent for inflammatory bowel diseases according to any one of claims 1 to 5, wherein the inflammatory bowel disease is ulcerative colitis.

8. A method for treating an inflammatory bowel disease, **characterized by** administering a compound represented by the formula (I): 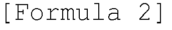 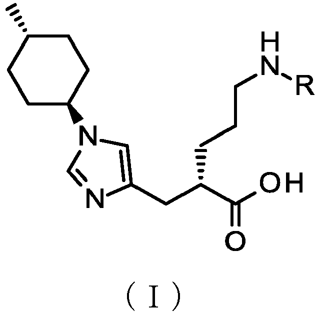 wherein R represents a hydrogen atom, a [(5-methyl-2-oxo-1,3-dioxol-4-yl)methoxy]carbonyl group, a [1-(isobutyryloxy)ethoxy]carbonyl group, a [1-(2,2-dimethylpropanoyloxy)ethoxy]carbonyl group, a {1-[(cyclohexylcarbonyl)oxy]ethoxy}carbonyl group, or a (1-acetoxyethoxy)carbonyl group,
or a pharmacologically acceptable salt thereof.

9. The method for treating an inflammatory bowel disease according to claim 8, wherein R represents a hydrogen atom.

10. The method for treating an inflammatory bowel disease according to claim 9, wherein the pharmacologically acceptable salt is p-toluenesulfonate.

11. The method for treating an inflammatory bowel disease according to claim 8, wherein R represents a [1-(isobutyryloxy)ethoxy]carbonyl group.

12. The method for treating an inflammatory bowel disease according to claim 11, wherein R represents a [(1R)-1-(isobutyryloxy)ethoxy]carbonyl group.

13. The method for treating an inflammatory bowel disease according to any one of claims 8 to 12, wherein the inflammatory bowel disease is ulcerative colitis, Crohn's disease, intestinal Behcet's disease, hemorrhagic rectal ulcer or pouchitis.

14. The method for treating an inflammatory bowel disease according to any one of claims 8 to 12, wherein the inflammatory bowel disease is ulcerative colitis.
